(19) Europäisches Patentamt
European Patent Office
Office européen des brevets

(11) Veröffentlichungsnummer: **0 403 812 B1**

(12) # EUROPÄISCHE PATENTSCHRIFT

(45) Veröffentlichungstag der Patentschrift: **12.07.95**

(21) Anmeldenummer: **90109736.0**

(22) Anmeldetag: **22.05.90**

Teilanmeldung 94102287.3 eingereicht am 22/05/90.

(51) Int. Cl.[6]: **C07D 241/02**, C07D 491/14, C07D 487/14, D06P 1/642, //(C07D491/14,307:00,307:00, 241:00),(C07D487/14,241:00, 209:00,209:00)

(54) **Diketopiperazin Derivate, deren Herstellung und deren Vervendung zum Färben von Kunststoffen.**

(30) Priorität: **03.06.89 DE 3918178**

(43) Veröffentlichungstag der Anmeldung: **27.12.90 Patentblatt 90/52**

(45) Bekanntmachung des Hinweises auf die Patenterteilung: **12.07.95 Patentblatt 95/28**

(84) Benannte Vertragsstaaten: **CH DE FR GB LI**

(56) Entgegenhaltungen:
**DE-A- 2 116 262**
**US-A- 3 407 203**

**TETRAHEDRON Band 30, 1974, Seiten 667-673; C. GALLINA et al.: "Condensation of 1,4-Diacetylpiperazine-2,5-Dione with Aldehydes"**

**CHEMICAL ABSTRACTS Band 80, Nr. 9, 4. März 1974, Seite 360, Zusammenfassung Nr. 47943b, Columbus, Ohio, US; CH.-G. SHIN et al.: "Alpha-beta-Unsaturated carboxylic acid derivatives. IV. General synthesis of unsaturated unsymmetric 3,6-disubstituted 2,5-piperazinediones" & Bull. Chem. Soc. Jap. 1973, Band 46, Nr. 12, Seiten 3876-3880**

(73) Patentinhaber: **BAYER AG**

**D-51368 Leverkusen (DE)**

(72) Erfinder: **Höchstetter, Hans Dr.**
**Färberstrasse 150**
**D-4000 Düsseldorf (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 403 812 B1

## Beschreibung

Die vorliegende Erfindung betrifft heterocyclische Verbindungen der unten angegebenen Formeln I und II, ein Verfahren zum Färben von Kunststoffen mit Verbindungen der Formel II und ein Verfahren zur Herstellung von Verbindungen der Formel II aus Verbindungen der Formel I.

Aus C.A. 47943 b (Band 80, 4.März 1974) ist ein allgemeiner Weg zur Synthese von ungesättigten, unsymmetrischen, 3,6-disubstituierten 2,5-Piperazindionen bekannt. Diese Verbindungen sind jedoch frei von Estergruppen in α-Stellung zu den exocyclischen Doppelbindungen. Gleiches trifft zu für die in Tetrahedron 30, 667-673 (1974) beschriebenen 3,6-diaryl-substituierten 2,5-Piperazindione.

Die in der US-A-3 407 203 beschriebenen Diketopiperazine sind weder ungesättigt, noch enthalten sie Estergruppen.

Schließlich beschreibt die DE-A-2 116 262 polymere Verbindungen, die ausgehend von bifunktionellen Diketopiperazinen zugänglich sind Die als Ausgangsprodukte beschriebenen Diketopiperazine enthalten keine exocyclischen Doppelbindungen.

Es wurden neue heterocyclische Verbindungen gefunden, die in einer ihrer tautomeren oder konfigurationsisomeren Formen der Formel

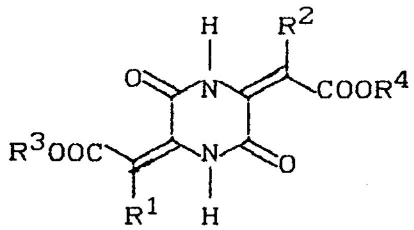

I

oder der Formel

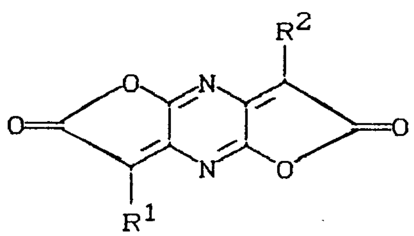

II

mit folgenden Substituentenbedeutungen entsprechen:

$R^1$, $R^2$ = Aryl, heterocyclischer Rest, der eine C = X-Bindung (X = C oder Heteroatom) in Konjugation zur exocyclischen Doppelbindung von I bzw. zur C = C-Bindung im Fünfring von II aufweist,

$R^3$, $R^4$ = H, Alkyl, Aryl, Cycloalkyl, oder Aralkyl,

wobei Aryl-, Aralkyl- und heterocycliche Reste gegebenenfalls durch Chlor, Brom und Fluor, -CN, $R^6$, $OR^7$, $SR^7$, $NR^8R^9$, $COOR^{10}$, $COR^{10}$, $NR^8COR^{10}$, $NR^8COOR^{10}$,$NR^8CONR^8R^9$, $NHSO_2R^{10}$, $SO_2R^{10}$, $SO_2OR^{10}$, $CONR^8R^9$, $SO_2NR^8R^9$, $N=N-R^{11}$, $OCOR^{10}$ und $OCONR^8R^9$ und Alkyl- und Cycloalkylreste gegebenenfalls durch Cl, Br, F, CN, $OCOR^{10}$, $OR^7$, $COOR^{10}$, $SR^7$, $CONR^8R^9$ und $OCONR^8R^9$ substituiert sein können, wobei

$R^6$ Alkyl oder Cycloalkyl bezeichnet,

$R^7$, $R^8$ und $R^9$ Wasserstoff, Alkyl, Cycloalkyl, Aralkyl, Aryl oder einen heterocyclischen Rest bezeichnen,

$R^8$ und $R^9$ auch zusammen unter Einschluß des N-Atoms einen 5- oder 6-gliedrigen heterocyclischen Ring bezeichnen können,

$R^{10}$ Wasserstoff, Alkyl, Cycloalkyl, Aralkyl oder Aryl,

$R^{11}$ den Rest einer Kupplungskomponente bezeichnet und Alkyl- und Cycloalkylreste $R^6$ gegebenenfalls durch Cl, Br, F, CN, $OCOR^{10}$, $OR^7$, $COOR^{10}$, $SR^7$, $CONR^8R^9$ und $OCONR^8R^9$,

Alkyl- und Cycloalkylreste $R^7$, $R^8$ und $R^9$ gegebenenfalls durch Cl, Br, F, CN, Mono-$C_1$-$C_4$-alkylamino, Di-$C_1$-$C_4$-alkylamino, Phenyl oder Naphthyl, die durch Cl, Br, F, $C_1$-$C_6$-Alkyl und $C_1$-$C_6$-Alkoxy substituiert sein können, oder durch heterocyclische Reste eines 5- oder 6-gliedrigen heterocyclischen Ringsystems mit 1 oder 2 Heteroatomen aus der Reihe O, N, S, an das ein Benzolring ankondensiert sein kann, die Aryl- und

Aralkylreste $R^8$ und $R^9$ gegebenenfalls durch Cl; Br, F, $C_1$-$C_{18}$-Alkyl oder $C_1$-$C_{18}$-Alkoxy und die Reste $R^{10}$ gegebenenfalls in gleicher Weise wie die Reste $R^8$ und $R^9$ substituiert sein können,

In den Formeln I und II ist $R^1$ vorzugsweise gleich $R^2$. Die Erfindung betrifft weiterhin Verfahren zur Herstellung der Verbindungen I und II sowie ihre Verwendung.

Alkyl steht vorzugsweise für $C_1$-$C_{18}$-Alkyl, insbesondere $C_1$-$C_4$-Alkyl. Cycloalkyl steht vorzugsweise für $C_3$-$C_7$-Cycloalkyl, insbesondere Cyclohexyl und Cyclopentyl.

Aralkyl steht insbesondere für Phenyl- und Naphthyl-$C_1$-$C_4$-alkyl, wobei die Arylreste, wie für Aryl angegeben, substituiert sein können.

Aryl steht vorzugsweise für solche carbocyclisch-aromatischen Reste, die 1, 2, 3 oder 4, insbesondere 1 oder 2 Ringe enthalten wie Phenyl, Diphenylyl und Naphthyl.

Als heterocyclische Reste stehen vorzugsweise solche heterocyclische (aromatische) Reste, die 1, 2, 3 oder 4, insbesondere 1 oder 2, fünf-, sechs- oder siebengliedrige Ringe enthalten, von denen mindestens einer 1, 2 oder 3, bevorzugt 1 oder 2 Heteroatome aus der Reihe O, N, S enthält. Als heterocyclische Reste seien beispielhaft genannt:
Pyridyl, Pyrimidyl, Pyrazinyl, Triazinyl, Furoyl, Pyrrolyl, Thiophenyl, Chinolyl, Cumarinyl, Benzfuranyl, Benzimidazolyl, Benzoxazolyl, Dibenzfuranyl, Benzothiophenyl, Dibenzothiophenyl, Indolyl, Carbazolyl, Pyrazolyl, Imidazolyl, Oxazolyl, Isoxazolyl, Thiazolyl, Indazolyl, Benzthiazolyl, Pyridazinyl, Cinnolyl, Chinazolyl, Chinoxalyl, Phthalazinyl, Phthalazindionyl, Phthalamidyl, Chromonyl, Naphtholactamyl, Chinolonyl, ortho-Sulfobenzoesäureimidyl, Maleinimidyl, Naptharidinyl, Benzimidazolonyl, Benzoxazolonyl, Benzthiazolonyl, Benzthiazothionyl, Chinazolonyl, Chinoxalonyl, Phthalazonyl, Dioxopyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Chinoxalindionyl, Benzoxazindionyl, Benzoxazinonyl und Naphthalimidyl.

$R^6$ bezeichnet vorzugsweise $C_1$-$C_{18}$-Alkyl, insbesondere $C_1$-$C_4$-Alkyl und vorzugsweise $C_3$-$C_7$-Cycloalkyl, insbesondere Cyclohexyl und Cyclopentyl.

$R^7$, $R^8$ und $R^9$ bezeichnen vorzugsweise Wasserstoff, $C_1$-$C_{18}$-Alkyl, insbesondere $C_1$-$C_4$-Alkyl, Cyclohexyl, Cyclopentyl, Phenyl- und Naphthyl-$C_1$-$C_4$-Alkyl, Phenyl und Naphthyl und einen Rest eines 5- oder 6-gliedrigen heterocyclischen Ringes mit 1, 2 oder 3 Heteroatomen aus der Reihe O, N, S, an den ein Benzolring ankondensiert sein kann.

$R^8$ und $R^9$ können zusammen unter Einschluß des N-Atoms, z.B. einen Morpholin-, Piperidin- oder Phthalimidring. Die Aryl- und Aralkylreste $R^8$ und $R^9$ können vorzugsweise durch Cl, Br, F, $C_1$-$C_4$-Alkyl oder $C_1$-$C_4$-Alkoxy substituiert sein.

$R^{10}$ bezeichnet vorzugsweise Wasserstoff, $C_1$-$C_{18}$-Alkyl, insbesondere $C_1$-$C_4$-Alkyl, Cyclopentyl, Cyclohexyl, Phenyl- und Naphthyl-$C_1$-$C_4$-alkyl, insbesondere Benzyl, Phenyl und Naphthyl.

$R^{11}$ bezeichnet vorzugsweise den Rest einer Kupplungskomponente aus der Benzol-, Naphthalin-, Acetessigsäurearylid-, Pyrazol- oder Pyridonreihe oder einen gegebenenfalls durch Cl, Br, F, $C_1$-$C_{18}$-Alkyl, vorzugsweise $C_1$-$C_4$-Alkyl und $C_1$-$C_{18}$-Alkoxy, vorzugsweise $C_1$-$C_4$-Alkoxy, substituierten Phenylrest.

Die Reste $R^3$ und $R^4$ in der Formel I können die Bedeutung von $R^8$ und $R^9$ annehmen.

Zur Herstellung von Verbindungen der Formel I setzt man literaturbekannte oder in Analogie zu literaturbekannten Verfahren herstellbare Glycinanhydridderivate der Formel

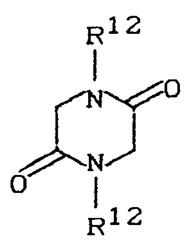

IV

mit Verbindungen der Formeln

$R^1$-COCOOR$^3$ V

und

$R^2$-COCOOR$^4$ VI

um. Der Rest $R^{12}$ kann Wasserstoff oder $COR^5$ bedeuten, wobei $R^1$ bis $R^5$ die oben angegebenen Bedeutungen besitzen.

Die Umsetzung erfolgt vorzugsweise in Gegenwart eines basischen Katalysators. Geeignet sind vorzugsweise aliphatische Amine, besonders tertiäre aliphatische Amine wie zum Beispiel Triethylamin.

Bevorzugt werden vom Katalysator und von Substrat V/VI äquimolare Mengen eingesetzt.

Die Reaktion wird vorzugsweise bei einer Temperatur von 30 bis 100 °C, besonders bevorzugt von 50 bis 70 °C durch-geführt.

Die Reaktion kann ohne Lösungsmittel durchgeführt werdenk Sie kann aber auch in Lösungsmitteln durchgeführt werdend Es eignen sich beispielsweise Alkohole oder dipolar aprotische Lösungsmittel wie Acetonitril, Dimethylsulfoxid, insbesondere Dimethylformamid.

Bei der erfindungsgemäßen Reaktion bilden sich meist alle drei möglichen Konfigurationsisomeren I a, I b und I c.

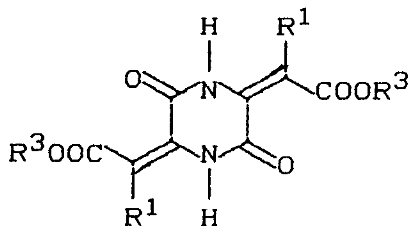

I a

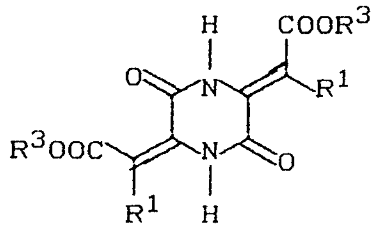

I b

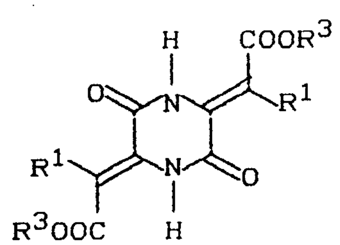

I c

Es ist möglich, die drei Isomeren chromatographisch zu trennen und einzeln zu charakterisieren.

Verbindungen der Formel II können hergestellt werden, indem man Piperazinderivate der Formel I in hochsiedenden Lösungsmitteln in Gegenwart eines geeigneten Katalysators oder in wasserentziehenden Mitteln wie Acetanhydrid oder Phosphoroxychlorid erhitzt. Geeignete Lösungsmittel sind beispielsweise o-Dichlorbenzol oder hochsiedende aromatische Kohlenwasserstoffe oder Ether, als Katalysator kommen p-Toluolsulfonsäure oder Schwefelsäure in Betracht. Die Reaktion erfolgt bei Temperaturen zwischen 120 und 240 °C, vorzugsweise 160 bis 180 °C.

Die Verbindungen der Formel I finden insbesondere als Zwischenprodukte zur Herstellung wertvoller Farbstoffe und Pigmente, insbesondere zur Herstellung von Verbindungen II, Verwendung.

Verbindungen der Formel II können als Farbstoffe oder als Zwischenprodukte zur Herstellung von Farbstoffen oder Pigmenten, insbesondere der Formel III, Verwendung finden. So können die Verbindungen der Formel II als lösliche Farbstoffe zur Kunststoffeinfärbung (Polystyrol) oder auch, gegebenenfalls nach vorheriger Einführung lipophiler Reste, als Dispersionsfarbstoffe verwendet werden. Auch Verbindungen der Formel I können zur Herstellung von Verbindungen der Formel III verwendet werden.

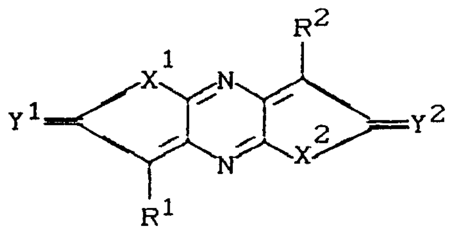

III

in der

$R^1$ und $R^2$ die bei den Formel I und II angegebene Bedeutung haben und

$X^1$, $X^2$, $Y^1$, $Y^2$ für O, S, NH, $NR^5$ stehen, wobei $X^1$ und $Y^1$ bzw. $X^2$ und $Y^2$ auch jeweils Teile eines ankondensierten heterocyclischen Fünf- oder Sechsrings bilden können, der dann vorzugsweise 2 N-Atome enthält und

$R^5$ für Wasserstoff, Alkyl, Aryl, Cycloalkyl, Aralkyl oder einen heterocyclischer Rest steht.

Verbindungen der Formel III werden hergestellt, indem man Verbindungen der Formel I oder II mit nahezu beliebigen primären Aminen in hochsiedenden Lösungsmitteln gegebenenfalls in Gegenwart eines geeigneten Katalysators unter Abspaltung von Wasser bzw. von Wasser und Alkohol umsetzt. Verbindungen der Formel II zeigen dabei eine ähnliche Reaktivität wie aromatische Carbonsäureanhydride, z.B. Perylentetracarbonsäuredianhydrid.

Wie bereits erwähnt, kann nahezu jedes primäre Amin eingesetzt werden, selbst sterisch stark gehinderte Amine wie 2,6-Diethyl-4-methylanilin oder sehr wenig basische Amine wie nitrogruppen- oder cyanogruppenhaltige aromatische Amine reagieren zu den Umsetzungsprodukten der Formel III. Niedrigsiedende aliphatische Amine können gegebenenfalls im Autoklaven umgesetzt werden

Verbindungen der Formel VII (siehe unten) mit $R^5$ = Wasserstoff werden hergestellt, indem man Verbindungen der Formel I in Formamid erhitzt.

Als hochsiedende Lösungsmittel zur Durchführung der erfindungsgemäßen Reaktion kommen z.B. in Frage: (chlorierte) aromatische Kohlenwasserstoffe wie Xylol, Chlorbenzol, Dichlorbenzole, Trichlorbenzole, Naphthalin oder Chlornaphthalin. Besonders geeignet ist Chinolin, Es kann aber auch Wasser (im Autoklaven) eingesetzt werden oder das betreffende Amin selbst als Lösungsmittel fungieren, sofern es einen ausreichend hohen Siedepunkt besitzt. Es kann sich als günstig erweisen, das entstehende Reaktionswasser azeotrop zu entfernen.

Die Reaktion läuft vorzugsweise bei Temperaturen von 130 bis 250 °C ab, besonders bevorzugt zwischen 180 und 210 °C.

Geeignete Katalysatoren sind Mineralsäuren, Carbonsäuren, Sulfonsäuren oder geeignete Metallsalze wie z.B. Zinkacetat oder Zinkchlorid.

Die Herstellung von Verbindungen der Formel III mit $X^1$, $X^2$ = S erfolgt durch Austausch der O-Atome in II gegen S, z,B, durch Schwefelung mit Lawessons-Reagens oder mit wasserfreiem $H_2S$.

Zur Herstellung von Verbindungen der Formel III, bei denen $X^1$ und $Y^1$ bzw, $X^2$ und $Y^2$ Teile eines ankondensierten heterocyclischen Fünf- oder Sechsrings mit 2 N-Atomen bilden, werden Verbindungen II mit primären aromatischen Diaminen, z.B. 1,8-Diaminonaphthalin oder o-Phenylendiaminen umgesetzt. Einen Vertreter dieses Verbindungstyps zeigt die folgende Formel:

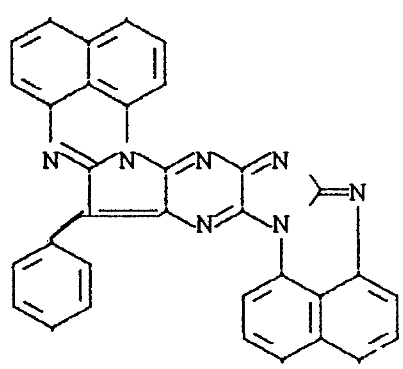

Bevorzugte Verbindungen der Formel III sind solche, die der Formel

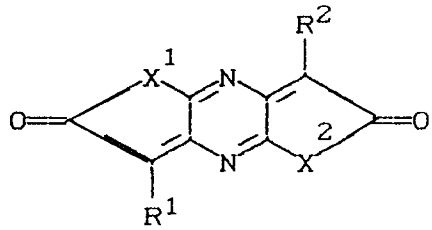

VI

und insbesondere der Formel

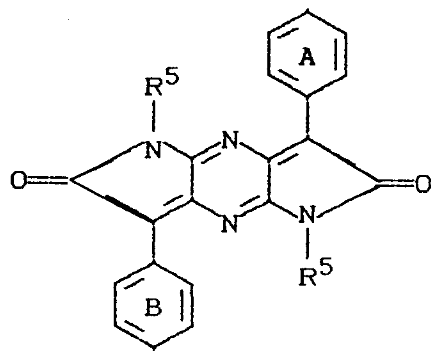

VII

entsprechen,
wobei die mit A und B bezeichneten Ringe, z.B. durch Halogen wie Cl, Br, F, -NHCOR[8] und/oder $-NR^8R^9$ substituiert sein können und wobei $R^1$, $R^2$, $X^1$, $X^2$, $R^5$, $R^8$ und $R^9$ die oben angegebenen Bedeutungen haben.

Die Verbindungen der Formel III fallen in einer für die Pigmentanwendung geeigneten Form an oder können durch an sich bekannte Nachbehandlungsverfahren in die geeignete Form überführt werden, z.B. durch Lösen oder Quellen in starken anorganischen Säuren wie Schwefelsäure und Austragen auf Eis, Die Feinverteilung kann auch durch Mahlen mit oder ohne Mahlhilfsstoffen wie anorganischen Salzen oder Sand, gegebenenfalls in Anwesenheit von Lösungsmitteln wie Toluol, Xylol, Dichlorbenzol oder N-Methylpyrrolidon erzielt werden, Farbstärke und Transparenz des Pigments können durch Variation der Nachbehandlung beeinflußt werden,

Die Verbindungen der Formel III eignen sich aufgrund ihrer Licht- und Migrationsechtheit für die verschiedensten Pigmentapplikationen. So können sie zur Herstellung von sehr echt pigmentierten Systemen in Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) oder mit Zement verstanden werden Zubereitungen sind z.B. Flushpasten mit organischen Flüssigkeiten oder Teige und Feinteige mit Wasser, Dispergiermitteln und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben.

Unter Druckfarben sind solche für den Papier-, Textil- und Blechdruck zu verstehen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylestern, Methacrylestern, Acrylnitril, Acrylamid, Butadien, Styrol sowie Polyrurethane und Polycarbonate. Die mit den beanspruchten Produkten pigmentierten Stoffe können auch in beliebiger Form vorliegen.

Die Pigmente der Formel III sind weiterhin ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig und in plastischen Massen gut verteilbar.

Beispiel 1

45,6 g bisacetyliertes Glycinanhydrid (IV, $R^{12}$ = $COCH_3$), 90,6 g Phenylglyoxylsäuremethylester und 89,8 g Triethylamin werden 8,5 Stunden lang bei 55 bis 60 °C gerührt. Danach wird das Triethylamin im Vakuum weitgehend abgezogen und der ölig-kristalline Rückstand mit 50 ml Methanol 12 Stunden lang verrührt. Dann kühlt man auf 0 °C ab, rührt noch 2 Stunden nach, saugt ab und wäscht mit eiskaltem Methanol. Man erhält 78,2 g des Isomerengemischs von

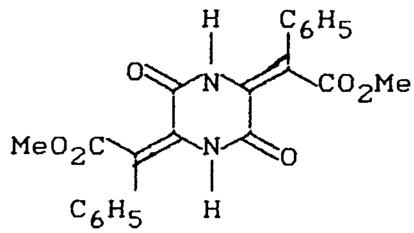

I d

als gelblich-weiße kristalline Substanz.

NMR (DMSO): $\delta$ = 3,64 (s, 6H, $OCH_3$), 7,15 - 7,35 (m, 1OH), 11,56 ppm (bs, 2H, NH): 1. Isomeres
$\delta$ = 3,68 (s, 3H, $OCH_3$), 3,73 (s, 3H, $OCH_3$), 7,15 - 7,38 (m, 1OH, Aromaten-H), 11,40 (bs, 1H, NH), 11,56 (bs, 1H, NH): 2. Isomeres
$\delta$ = 3,66 (s, 6H, $OCH_3$), 7,38 - 7,52 (m, 1OH, Aromaten-H), 10,20 ppm (bs, 2H, NH): 3. Isomeres

Erfindungsgemäße Verbindungen I mit $R^3$, $R^4$ = H werden hergestellt, indem man Verbindungen der Formel I mit $R^3$, $R^4$ ≠ H bei Temperaturen von 40 bis 80 °C, vorzugsweise 50 bis 60 °C, in 96-prozentiger Schwefelsäure löst und danach unter Kühlung durch Verdünnen mit Wasser wieder ausfällt, Auch diese Carbonsäurederivate stellen wertvolle Zwischenprodukte zur Herstellung organischer Farbstoffe und Pigmente dar.

Ganz analog zu Beispiel 1 kann die Umsetzung auch mit 4-Chlorphenylglyoxylsäuremethylester durchgeführt werden, was zu einem Isomerengemisch

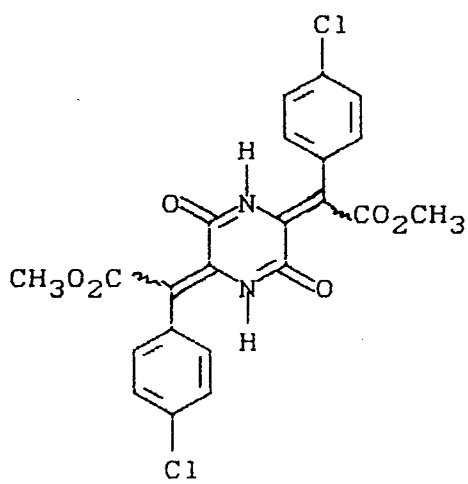

I e

führt.

Beispiel 2

10 g des in Beispiel 1 erhaltenen Isomerengemisches I d werden in 50 ml o-Dichlorbenzol in Gegenwart von 0,1 g p-Toluolsulfonsäure 8 Stunden lang am Rückfluß gekocht. Nach Abkühlen wird abgesaugt und mit Methanol gewaschen. Man erhält 6 g eines schwarzvioletten Kristallpulvers.

UV (DMF): $\lambda_{max}$ = 488 nm (41 800)

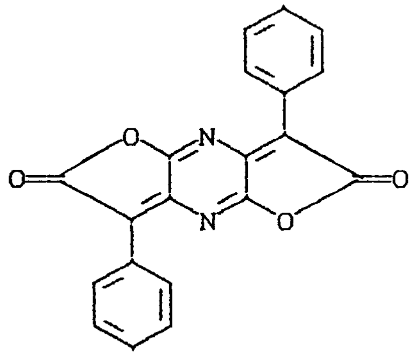

Einarbeiten dieser Substanz in Polystyrol ergibt eine orangerote Färbung.

Beispiel 3

6,5 g des in Beispiel 1 hergestellten Isomerengemisches I d werden in 20 ml Formamid 4 Stunden lang bei 150 °C gerührt. Nach Verrühren mit 20 ml Methanol werden 4 g der Verbindung

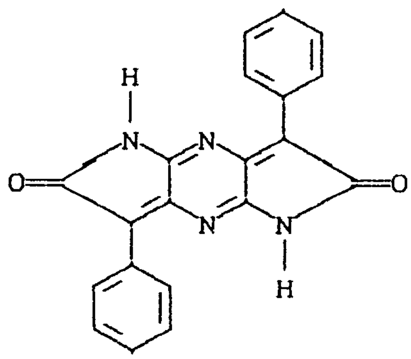

$\lambda_{max}$ = 471 nm (25 600)
isoliert, welche in Polystyrol eingearbeitet eine rotorange Färbung ergibt.

Beispiel 4

10 g des nach Beispiel 1 hergestellten Isomerengemisches I d werden zusammen mit 6,3 g 4-Chloranilin und 100 mg p-Toluolsulfonsäure in 50 ml o-Dichlorbenzol 12 Stunden lang am Rückfluß gekocht. Nach Absaugen bei Raumtemperatur isoliert man 10 g der Verbindung

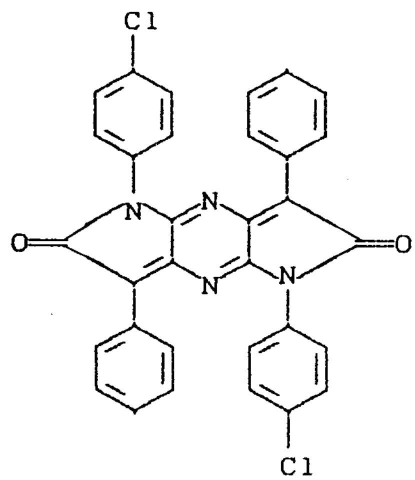

$\lambda_{max}$ = 484 nm
welche ein blaustichig rotes Pigment darstellt.

Beispiel 5

10,3 g des nach Beispiel 1 hergestellten Isomerengemisches I d werden zusammen mit 13,0 g 4-Aminoazobenzol und 0,1 g Zinkacetat in 100 ml Chinolin 2 Stunden lang bei 190 bis 195 °C gerührt. Nach Abkühlen auf 65 °C wird mit 100 ml Ethanol verrührt und abgesaugt. Man isoliert 12 g eines braunroten Pigments der Formel

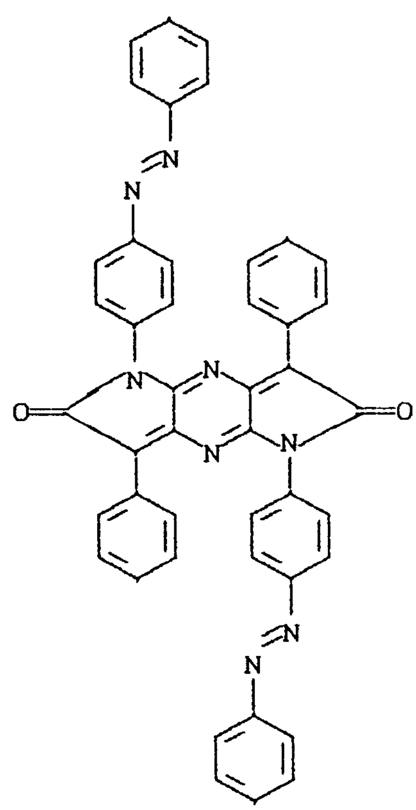

Statt des Isomerengemisches I d kann auch jeweils das Isomerengemisch I e (4-Chlorphenylderivat) eingesetzt werden.

Die in der folgenden Tabelle 1 aufgeführten Farbstoffe und Pigmente werden analog zu den Arbeitsweisen der Beispiele 3 - 5 erhalten.

Tabelle 1

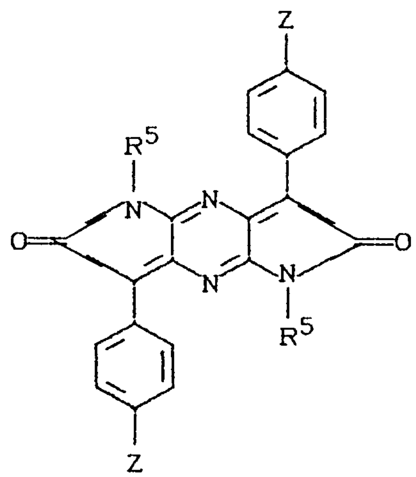

| Bsp. Nr. | Z | $R^5$ | Bemerkungen |
|---|---|---|---|
| 6 | H | n-Butyl | braunroter, löslicher Farbstoff |
| 7 | Cl | n-Butyl | braunroter, löslicher Farbstoff |
| 8 | H | $CH_3$ | braunroter, löslicher Farbstoff |
| 9 | Cl | $CH_3$ | braunroter, löslicher Farbstoff |
| 10 | H | Hexadecyl | roter Farbstoff |
| 11 | H | $-CH_2-CH_2-OH$ | roter Farbstoff |
| 12 | H | 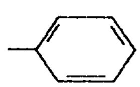 | roter Farbstoff |
| 13 | Cl | 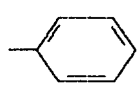 | rotes Pigment |
| 14 | H | 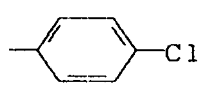 | rotviolettes Pigment |

| Bsp. Nr. | Z | $R^5$ | Bemerkungen |
|---|---|---|---|
| 15 | Cl | 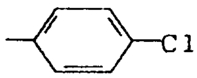 | rotvioletes Pigment |
| 16 | H | 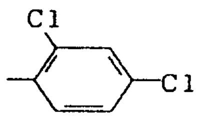 | orangerotes Pigment |
| 17 | Cl | 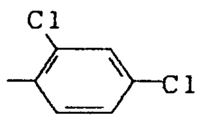 | orangerotes Pigment |
| 18 | H | 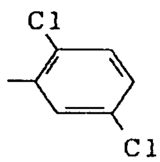 | braunrotes Pigment |
| 19 | H | 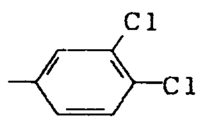 | braunrotes Pigment |
| 20 | Cl | 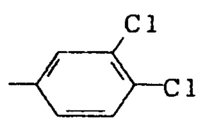 | braunrotes Pigment |
| 21 | H | 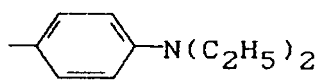 | roter Farbstoff |
| 22 | Cl | 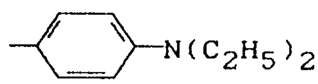 | roter Farbstoff |
| 23 | H | 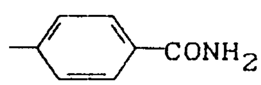 | rotviolettes Pigment |

| Bsp. Nr. | Z | $R^5$ | Bemerkungen |
|---|---|---|---|
| 24 | Cl | 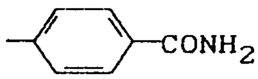 | rotviolettes Pigment |
| 25 | H | 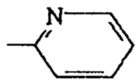 | roter Farbstoff |
| 26 | Cl | 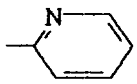 | roter Farbstoff |
| 27 | H | 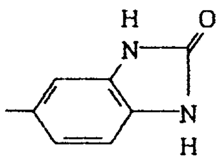 | braunrotes Pigment |
| 28 | Cl | 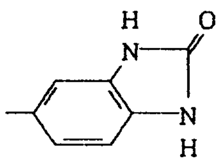 | braunrotes Pigment |
| 29 | H | 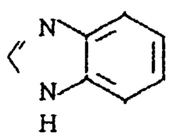 | braunrotes Pigment |
| 30 | Cl | 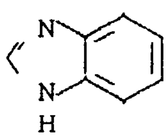 | braunrotes Pigment |
| 31 | H | 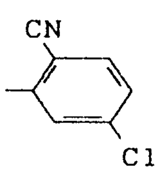 | rotes Pigment |

| Bsp. Nr. | Z | $R^5$ | Bemerkungen |
|---|---|---|---|
| 32 | Cl | 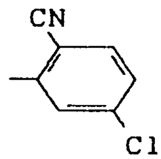 | rotes Pigment |
| 33 | H | 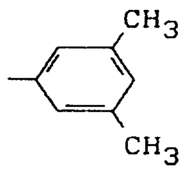 | roter Farbstoff |
| 34 | Cl | 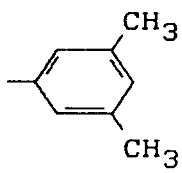 | roter Farbstoff |
| 35 | H | 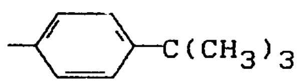 | roter Farbstoff |
| 36 | Cl | 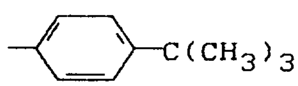 | roter Farbstoff |
| 37 | H | 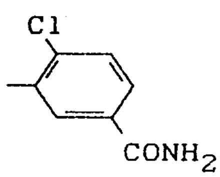 | braunrotes Pigment |
| 38 | Cl | 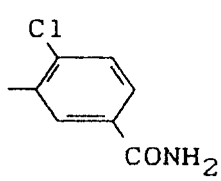 | braunrotes Pigment |

| Bsp. Nr. | Z | $R^5$ | Bemerkungen |
|---|---|---|---|
| 39 | H | 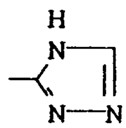 | rotoranges Pigment |
| 40 | Cl | 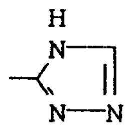 | rotoranges Pigment |
| 41 | H | 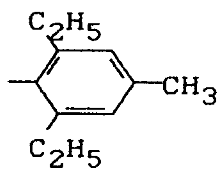 | roter Farbstoff |
| 42 | Cl | 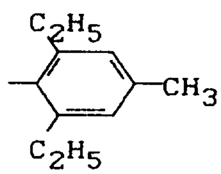 | roter Farbstoff |
| 43 | H | 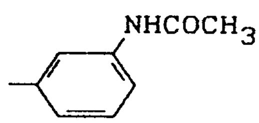 | braunrotes Pigment |
| 44 | Cl | 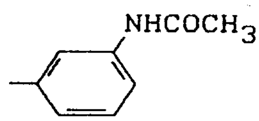 | braunrotes Pigment |
| 45 | H | 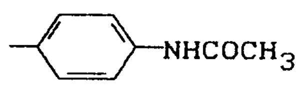 | braunrotes Pigment |
| 46 | Cl | 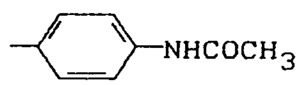 | braunrotes Pigment |
| 47 | H | 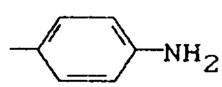 | roter Farbstoff |

| Bsp. Nr. | Z | $R^5$ | Bemerkungen |
|---|---|---|---|
| 48 | Cl | 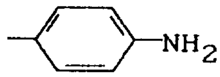 | roter Farbstoff |
| 49 | H | 1-Anthrachinonyl | rotes Pigment |
| 50 | Cl | 1-Anthrachinonyl | rotes Pigment |
| 51 | H | 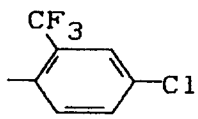 | orangeroter Farbstoff |
| 52 | Cl | 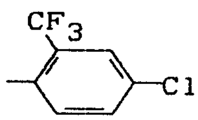 | orangeroter Farbstoff |
| 53 | H | 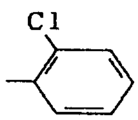 | oranger Farbstoff |
| 54 | Cl | 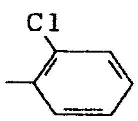 | oranger Farbstoff |
| 55 | H | 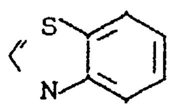 | schwarzbraunes Pigment |
| 56 | Cl | 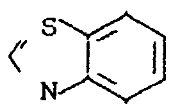 | schwarzbraunes Pigment |
| 57 | H | 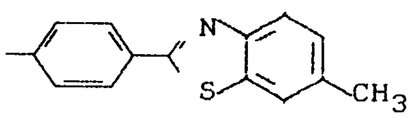 | braunes Pigment |
| 58 | Cl | 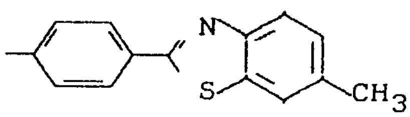 | braunes Pigment |

| Bsp. Nr. | Z | $R^5$ | Bemerkungen |
|---|---|---|---|
| 59 | H | 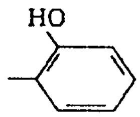 | braunroter Farbstoff |
| 60 | Cl | 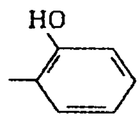 | braunroter Farbstoff |
| 61 | H | 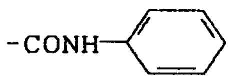 | braunroter Farbstoff |
| 62 | Cl | 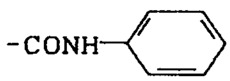 | braunroter Farbstoff |
| 63 | H | 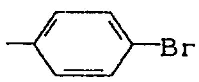 | rotbraunes Pigment |
| 64 | Cl | 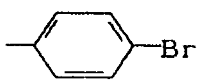 | rotbraunes Pigment |
| 65 | H | 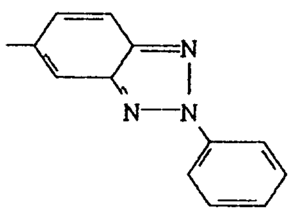 | braunes Pigment |
| 66 | Cl | 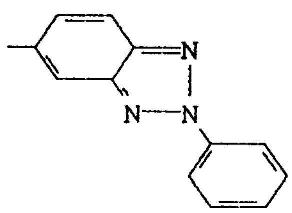 | braunes Pigment |

| Bsp. Nr. | Z | $R^5$ | Bemerkungen |
|---|---|---|---|
| 67 | H | 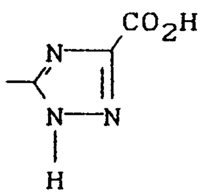 | rotes Pigment |
| 68 | Cl | 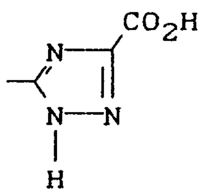 | rotes Pigment |
| 69 | H | 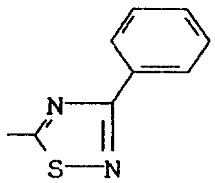 | rotes Pigment |
| 70 | Cl | 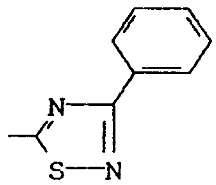 | rotes Pigment |
| 71 | H | 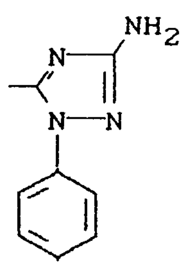 | rotes Pigment |
| 72 | Cl | 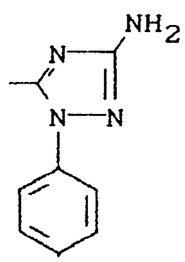 | rotes Pigment |

| Bsp. Nr. | Z | $R^5$ | Bemerkungen |
|---|---|---|---|
| 73 | H | 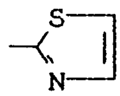 | roter Farbstoff |
| 74 | Cl | 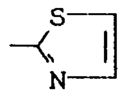 | roter Farbstoff |
| 75 | H | 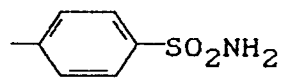 | roter Farbstoff |
| 76 | Cl | 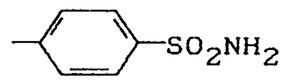 | roter Farbstoff |

<u>Beispiel 77</u> (Anwendungsbeispiel)

4 g feingemahlenes Pigment gemäß Beispiel 5 werden in 92 g eines Einbrennlackes folgender Zusammensetzung dispergiert:

33 % Alkydharz
15 % Melaminharz
5 % Glykolmonomethylether
34 % Xylol
13 % Butanol

Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden. Nach erfolgter Dispergierung wird der pigmentierte Lack auf Papier-, Glas-, Kunststoff- oder Metall-Folien aufgetragen und 30 Minuten bei 130 °C eingebrannt. Die Lackierungen besitzen sehr gute Licht- und Wetterbeständigkeit sowie gute Überlackierechtheit.

Der Einbrennlack, hergestellt gemäß Beispiel 77, wird auf weißes Papier aufgestrichen und bei 130 °C eingebrannt.

<u>Beispiel 78</u> (Anwendungsbeispiel)

0,2 g Pigment nach Beispiel 5 werden mit 100 g Polyethylen-, Polypropylen oder Polystyrolgranulat gemischt. Die Mischung kann entweder bei 220 bis 280 °C direkt in einer Spritzgußmaschine verspritzt oder in einer Strangpresse zu gefärbten Stäben bzw. auf dem Mischwalzwerk zu gefärbten Fellen verarbeitet werden. Die Stäbe bzw. Felle werden gegebenenfalls granuliert und in einer Spritzgußmaschine verspritzt.

Die roten Formlinge besitzen sehr gute Licht- und Migrationsechtheit. In ähnlicher Weise können bei 280 bis 300 °C, gegebenenfalls unter Stickstoffatmosphäre, synthetische Polyamide aus Caprolactam oder Adipinsäure und Hexamethylendiamin oder die Kondensate aus Terephthalsäure und Ethylenglykol gefärbt werden.

## Patentansprüche

1. Verbindungen, die in einer ihrer tautomeren oder konfigurationsisomeren Formen der Formel

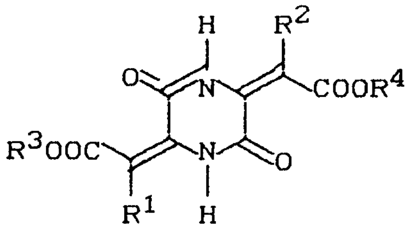

I

entsprechen, in der

$R^1$, $R^2$ Aryl oder einen heterocyclischen Rest, der eine C = X-Bindung (X = C oder Heteroatom) in Konjugation zur exocyclischen Doppelbindung von I aufweist und

$R^3$, $R^4$ = H, Alkyl, Aryl, Cycloalkyl oder Aralkyl bezeichnen,

wobei Aryl-, Aralkyl- und heterocycliche Reste gegebenenfalls durch Chlor, Brom und Fluor, -CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N = N\text{-}R^{11}$, $OCOR^{10}$ und $OCONR^8R^9$ und Alkyl- und Cycloalkylreste gegebenenfalls durch Cl, Br, F, CN, $OCOR^{10}$, $OR^7$, $COOR^{10}$, $SR^7$, $CONR^8R^9$ und $OCONR^8R^9$ substituiert sein können,

wobei

$R^6$ Alkyl oder Cycloalkyl bezeichnet,

$R^7$, $R^8$ und $R^9$ Wasserstoff, Alkyl, Cycloalkyl, Aralkyl, Aryl oder einen heterocyclischen Rest bezeichnen,

$R^8$ und $R^9$ auch zusammen unter Einschluß des N-Atoms einen 5- oder 6-gliedrigen heterocyclischen Ring bezeichnen können,

$R^{10}$ Wasserstoff, Alkyl, Cycloalkyl, Aralkyl oder Aryl,

$R^{11}$ den Rest einer Kupplungskomponente bezeichnet und Alkyl- und Cycloalkylreste $R^6$ gegebenenfalls durch Cl, Br, F, CN, $OCOR^{10}$, $OR^7$, $COOR^{10}$, $SR^7$, $CONR^8R^9$ und $OCONR^8R^9$,

Alkyl- und Cycloalkylreste $R^7$, $R^8$ und $R^9$ gegebenenfalls durch Cl, Br, F, CN, Mono-$C_1$-$C_4$-alkylamino, Di-$C_1$-$C_4$-alkylamino, Phenyl oder Naphthyl, die durch Cl, Br, F, $C_1$-$C_6$-Alkyl und $C_1$-$C_6$-Alkoxy substituiert sein können, oder durch heterocyclische Reste eines 5- oder 6-gliedrigen heterocyclischen Ringsystems mit 1 oder 2 Heteroatomen aus der Reihe O, N, S, an das ein Benzolring ankondensiert sein kann, die Aryl- und Aralkylreste $R^8$ und $R^9$ gegebenenfalls durch Cl, Br, F, $C_1$-$C_{18}$-Alkyl oder $C_1$-$C_{18}$-Alkoxy und

die Reste $R^{10}$ gegebenenfalls in gleicher Weise wie die Reste $R^8$ und $R^9$ substituiert sein können,

2. Verbindungen der Formel

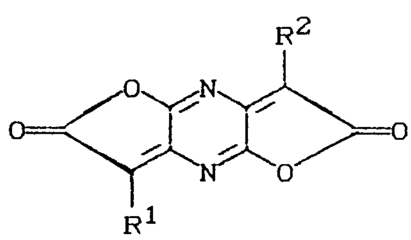

II

in der

$R^1$ und $R^2$ die im Anspruch 1 angegebene Bedeutung haben, wobei ein heterocyclischer Rest mit einer C = X-Bindung eine Konjugation zur C = C-Bindung im Fünfring von II aufweist.

3. Verfahren zum Färben von Kunststoffen, dadurch gekennzeichnet, daß man Verbindungen gemäß Anspruch 2 verwendet.

**4.** Verfahren zur Herstellung von Verbindungen gemäß Anspruch 2, dadurch gekennzeichnet, daß man Verbindungen gemäß Anspruch 1 als Zwischenprodukte verwendet.

## Claims

**1.** Compounds which, in one of their tautomeric or configuration isomer forms correspond to the formula

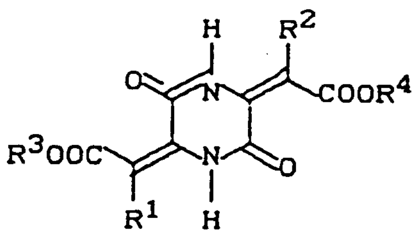

I

in which

$R^1$ and $R^2$ designate aryl or a heterocyclic radical which contains a C=X bond (X = C or a hetero atom) in conjugation with the exocyclic double bond of I and

$R^3$ and $R^4$ designate H, alkyl, aryl, cycloalkyl or aralkyl,

wherein aryl, aralkyl and heterocyclic radicals can optionally be substituted by chlorine, bromine and fluorine, -CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N=N-R^{11}$, $OCOR^{10}$ and $OCONR^8R^9$ and alkyl and cycloalkyl radicals can optionally be substituted by Cl, Br, F, CN, $OCOR^{10}$, $OR^7$, $COOR^{10}$, $SR^7$, $CONR^8R^9$ and $OCONR^8R^9$,wherein

$R^6$ designates alkyl or cycloalkyl,

$R^7$, $R^8$ and $R^9$ designate hydrogen, alkyl, cycloalkyl, aralkyl, aryl or a heterocyclic radical,

$R^8$ and $R^9$ can also designate together, including the N atom, a 5- or 6-membered heterocyclic ring,

$R^{10}$ designates hydrogen, alkyl, cycloalkyl, aralkyl or aryl,

$R^{11}$ designates the radical of a coupling component, and alkyl and cycloalkyl radicals $R^6$ can optionally be substituted by Cl, Br, F, CN, $OCOR^{10}$, $OR^7$, $COOR^{10}$, $SR^7$, $CONR^8R^9$ and $OCONR^8R^9$,

alkyl and cycloalkyl radicals $R^7$, $R^8$ and $R^9$ can optionally be substituted by Cl, Br, F, CN, mono-$C_1$-$C_4$-alkylamino, di-$C_1$-$C_4$-alkylamino, phenyl or naphthyl, which can be substituted by Cl, Br, F, $C_1$-$C_6$-alkyl and $C_1$-$C_6$-alkoxy, or by heterocyclic radicals of a 5- or 6-membered heterocyclic ring system with 1 or 2 hetero atoms from the series consisting of O, N and S, onto which ring system a benzene ring can be fused, the aryl and aralkyl radicals $R^8$ and $R^9$ can optionally be substituted by Cl, Br, F, $C_1$-$C_{18}$-alkyl or $C_1$-$C_{18}$-alkoxy and

the radicals $R^{10}$ can optionally be substituted in the same manner as the radicals $R^8$ and $R^9$.

**2.** Compounds of the formula

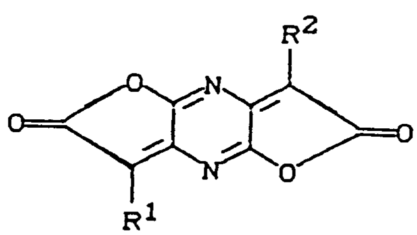

II

in which

$R^1$ and $R^2$ have the meaning given in Claim 1, wherein a heterocyclic radical with a C=X bond is in conjugation with the C=C bond in the five-membered ring of II.

**3.** Process for dyeing plastics, characterized in that compounds according to Claim 2 are used.

4. Process for the preparation of compounds according to Claim 2, characterized in that compounds according to Claim 1 are used as intermediate products.

**Revendications**

1. Composés qui répondent sous l'une de leurs formes tautomères ou d'isomères de configuration à la formule 1

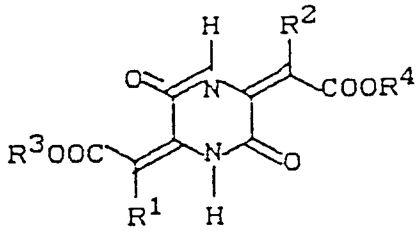

I

dans laquelle,

$R^1{}_1$ $R^2$ représentent un aryle ou un reste hétérocyclique, qui présente une liaison C=X (X = C ou hétéroatome) en conjugaison à une double liaison exocyclique de I et

$R^3$, $R^4$ représentent H, alkyle, aryle, cycloalkyle ou aralkyle,

dans laquelle les restes aryles, aralkyles et hétérocycliques peuvent être substitués le cas échéant par du chlore, du brome et du fluor, -CN, $R^6$, $OR^7$, $SR^7$, $NR^8R^9$, $COOR^{10}$, $COR^{10}$, $NR^8COR^{10}$, $NR^8COOR^{10}$, $NR^8CONR^8R^9$, $NHSO_2R^{10}$, $SO_2R^{10}$, $SO_2OR^{10}$, $CONR^8R^9$, $SO_2NR^8R^9$, $N=N\text{-}R^{11}$, $OCOR^{10}$ et $OCONR^8R^9$ et les restes alkyles et cycloalkyles peuvent être substitués le cas échéant par Cl, Br, F, CN, $OCOR^{10}$, $OR^7$, $COOR^{10}$, $SR^7$, $CONR^8R^9$ et $OCONR^8R^9$,

dans lesquels $R^6$ représente un alkyle ou cycloalkyle,

$R^7$, $R^8$ et $R^9$ représentent un hydrogène, alkyle, cycloalkyle, aralkyle, aryle ou un reste hétérocyclique,

$R^8$ et $R^9$ peuvent représenter aussi ensemble en incluant l'atome de N un cycle hétérocyclique à 5 ou 6 maillons,

$R^{10}$ représente un hydrogène, alkyle, cycloalkyle, aralkyle ou aryle

$R^{11}$ représente un reste d'un composant de copulation et des restes alkyles et cycloalkyles $R^6$ substitués le cas échéant par Cl, Br, F, CN, $OCOR^{10}$, $OR^7$, $COOR^{10}$, $SR^7$, $CONR^8R^9$ et $OCONR^8R^9$,

des restes alkyles et cycloalkyles $R^7$, $R^8$ et $R^9$ peuvent être substitués le cas échéant par Cl, Br, F, CN, monoalkylamino $C_1$-$C_4$, dialkylamino $C_1$-$C_4$, phényle ou naphtyle, qui peuvent être substitués par Cl, Br, F, alkyle $C_1$-$C_6$ ou alcoxy $C_1$-$C_6$, ou par des restes hétérocycliques d'un système cyclique hétérocyclique à 5 ou 6 maillons avec un ou deux hétéroatomes de la série O, N, S, sur lequel peut être condensé un noyau benzénique, les restes aryles et aralkyles $R^8$ et $R^9$ peuvent être substitués le cas échéant par Cl, Br, F, alkyle $C_1$-$C_{18}$ ou alcoxy $C_1$-$C_{18}$ et les restes $R^{10}$ peuvent être substitués le cas échéant de la même façon que les restes $R^8$ et $R^9$.

2. Composés de formule :

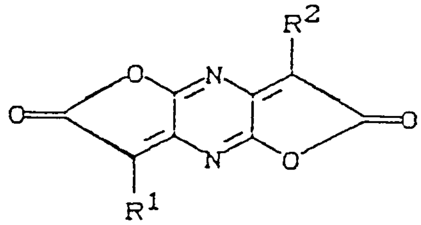

II

dans laquelle

$R^1$ et $R^2$ ont les significations données à la revendication 1, dans laquelle un reste hétérocyclique avec une liaison C=X présente une conjugaison vis-à-vis de la liaison C=C dans le cycle à 5 maillons de II.

**3.** Procédé de coloration de matières plastiques, caractérisé en ce qu'on utilise des composés selon la revendication 2.

**4.** Procédé de préparation de composés selon la revendication 2, caractérisé en ce qu'on utilise des composés selon la revendication 1 comme produits intermédiaires.